# EUROPEAN PATENT SPECIFICATION

(11) **EP 4 510 973 B1**
(45) Date of publication and mention of the grant of the patent: **06.05.2026**
(21) Application number: 23738249.4
(22) Date of filing: 02.06.2023
(51) Int. Cl.: A61F 2/24

(54) **MAGNETIC RELEASE MECHANISM**
MAGNETISCHER AUSLÖSEMECHANISMUS
MÉCANISME À LIBÉRATION MAGNÉTIQUE

(30) Priority: 06.06.2022 US 202263349475 P
(43) Date of publication of application: 26.02.2025
(73) Proprietor: Edwards Lifesciences Corporation, Irvine, CA 92614 (US)
(72) Inventor: VALDEZ, Michael G., Irvine, CA 92614 (US)
(74) Representative: Venner, Julia Ann
(86) International application number: PCT/US2023/024225
(87) International publication number: WO 2023/239595

(56) References cited:
- EP-A1- 2 749 309
- US-A1- 2015 112 377

## Description

### CROSS-REFERENCE TO RELATED APPLICATION(S)

This application claims the benefit of U.S. Provisional Application No. 63/349,475, filed June 6, 2022, and entitled "MAGNETIC RELEASE MECHANISM." .

### BACKGROUND

The patent disclosure relates generally to mechanisms for delivering an implant to a patient, and more specifically to an implant delivery system utilizing a catheter with a magnetic release mechanism.

Minimally invasive surgical techniques are improving patient outcomes and recovery times. Catheters can be used to attach sensors and mechanical couplings among other possible medical device implants within a patient during some minimally invasive procedures. Prior attempts to deliver implants includes catheters equipped with a mechanical coupler (e.g., grips, clamps, and other holding attachments and adapters). Each mechanical coupler incorporates complex mechanisms which require larger catheter sizes to facilitate integration of the mechanical coupler into the catheter. Furthermore, mechanical coupling mechanisms can over restrain the implant, particularly in a torsional direction, which can lead to inadvertent tissue damage when force or moments applied by the catheter exceed tissue strength.

EP 2 749 309 describes an endoluminal prosthesis (10) for placing in a body passage of a patient, including a ureteral stent (12), the ureteral stent comprising a generally tubular housing (14) having a proximal end (16) and a distal end (18) and a lumen (20) longitudinally disposed therethrough, with cation-exchange resin beads (22) disposed within the tubular housing, and at least one anchoring mechanism (24) disposed on a distal end of the tubular housing, where at least one retention screen (26) is disposed within the lumen of the ureteral stent configured to retain the plurality of beads. US 2015/112377 describes a collapsible blood filtering aortic arch bridge comprising a dumbbell shaped chassis having a tubular waist, a first conical end, and a second conical end such that only a periphery of the first and second ends contact the intima of an aortic arch when the bridge is disposed and expanded within the aortic arch of a patient. The waist is flexible so that the bridge can bend to comply with the curvature of the aortic arch. The bridge additionally comprises a blood filtering sleeve disposed over an interior or an exterior of the chassis for filtering blood flowing through the bridge into aortic arch vessels of the patient when the bridge is disposed within the aortic arch. Furthermore, the bridge comprises a retrieval sleeve disposed over the exterior of the chassis for collapsing the bridge to a cylindrical form for retrieval of the bridge from the aortic arch.

### SUMMARY

A catheter includes a magnetic release mechanism comprising a magnet. The catheter includes a shaft that defines a lumen. The shaft extends from a proximal end to a distal end. A spacer wall within a distal region of the shaft encloses the lumen, and the magnet abuts the spacer wall between the spacer wall and the proximal end of the catheter.

A catheterization procedure, not forming part of the presently claimed invention, includes guiding the catheter to an attachment point and rotating a handle of the catheter to thereby rotate the magnet. Rotation of the magnet induces rotation of an implant retained at the distal end of the catheter by the magnet. Disengaging the magnetic release mechanism decouples the implant from the catheter.

The invention is directed to a catheter as defined in independent claim 1.

### BRIEF DESCRIPTION OF THE DRAWINGS

FIG. 1A is a schematic view of a catheter with a first exemplary magnetic release mechanism.
FIG. 1B is a schematic side view illustrating exemplary features of an implant.
FIG. 1C is an end view illustrating exemplary features of the implant.
FIG. 1D and FIG. 1E are schematic views of the proximal and distal ends of the catheter depicted in FIG. 1A.
FIG. 1F is a schematic view of the catheter in which the magnetic release mechanism is disengaged.
FIG. 2A is a schematic view of a catheter equipped with a second exemplary magnetic release mechanism.
FIG. 2B and FIG. 2C are schematic views of the proximal and distal ends of the catheter depicted in FIG. 2A.
FIG. 3A, FIG. 3B, and FIG. 3C are schematic views of a patient's heart depicting attachment of an implant utilizing a catheter equipped with a magnetic release mechanism.
FIG. 4 is a flow chart describing a procedure for attaching an implant to a patient.

### DETAILED DESCRIPTION

As described herein, a catheter includes a magnetic release mechanism housed within a distal region of a lumen. Catheters equipped with the magnetic release mechanism can be used to deliver and/or manipulate an implant to an attachment site within the patient to perform minimally invasive procedures. The magnetic release mechanism in accordance with this disclosure is less complex relative to prior mechanical release mechanisms, which in turn reduces the minimum possible catheter size. Additionally, the magnetic release mechanism can also limit force imparted to the patient's tissue by decoupling from the implant at a desired limiting force or torque.

As used herein, the catheter, or components thereof, may be described as having a proximal and/or a distal portion, end, or region. Proximal portions, ends, or regions of catheters and/or its components refer to portions, ends, or regions of components and catheters positioned towards the exterior of the patient. For instance, the proximal end of a catheter can be manipulated by a practitioner to guide the catheter to an implant attachment site. Moreover, distal portions, ends, or regions of catheters or its components refer to portions of the catheter or its components positioned towards the interior of the patient.

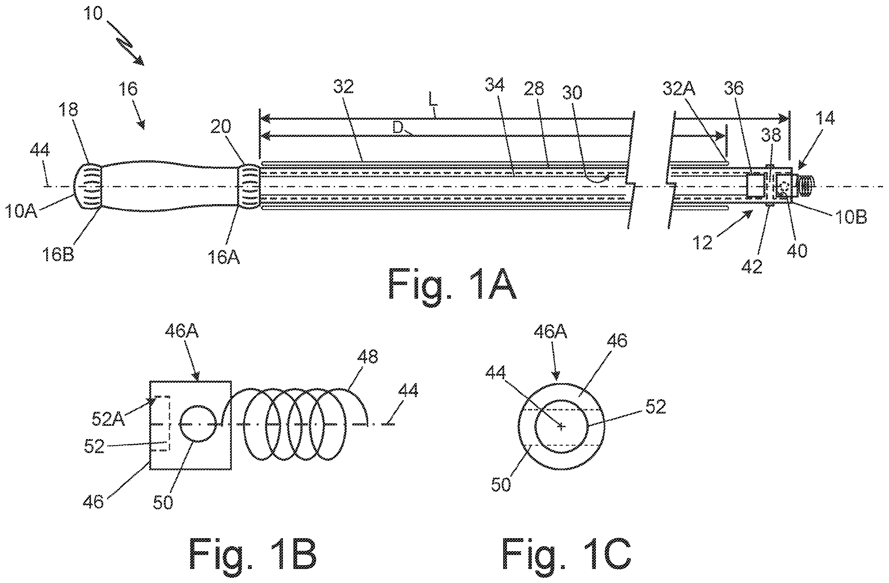

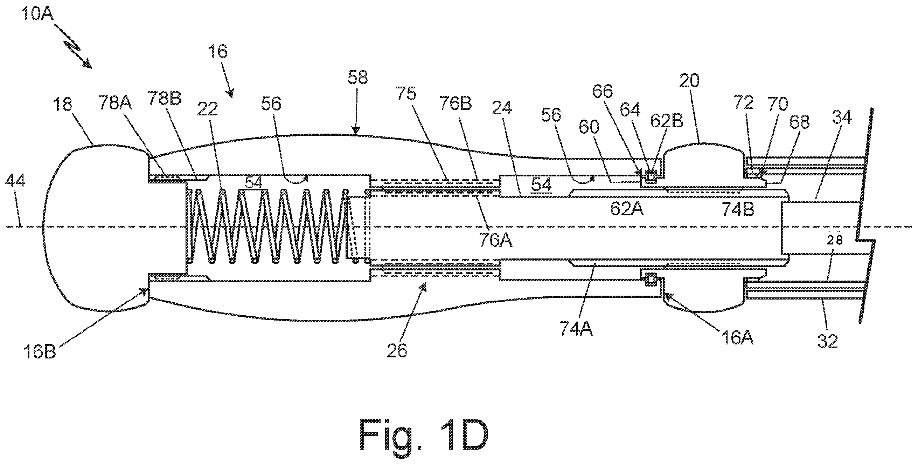

FIG. 1A is a schematic depicting catheter 10 equipped with magnetic release mechanism 12 operable to attach, remove, or adjust implant 14 within a patient without imparting excessive force into the patient's tissue. At proximal end 10A, catheter 10 includes handle 16, end cap 18, and knob 20 that enclose spring 22, terminal member 24, and coupling 26 shown in FIG. 1D. Extending from proximal end 10A towards distal end 10B, catheter 10 includes shaft 28, lumen 30, sheath 32, and inner shaft 34. At distal end 10B, catheter 10 includes magnet 36, spacer wall 38, socket 40, marker strip 42, and implant 14. Magnetic release mechanism 12 is formed by magnet 36, inner shaft 34, terminal member 24, knob 20, shaft 28, lumen 30, and spacer wall 38.

The length L of catheter 10 is measured longitudinally along shaft 28 from knob 20 to distal end 10B of catheter 10. The longitudinal dimension of catheter 10 and its subcomponents extends along centerline 44. Catheter length can be adapted to the intended insertion site of catheter 10 and attachment site of implant 14. For instance, central venous catheters intended for insertion through the internal jugular vein or subclavian vein can be shorter than central venous catheters inserted through the common femoral vein. Typically, length L of catheter 10 can be between 60 cm and 110 cm. However, catheter 10 can have shorter or longer length L as required.

Shaft 28 is a flexible tubular member extending from knob 20 to distal end 10B of catheter 10. Shaft 28 forms lumen 30, which extends from a proximal end to a distal end of shaft 28, or from a proximal end of shaft 28 to spacer wall 38. Where shaft 28 includes a single lumen 30, shaft 28 has an annular cross-section taken normal to centerline 44. However, magnetic release mechanism 12 can be incorporated into a catheter with multiple lumens. If shaft 28 includes multiple lumens 30, one or more interior walls partition an exterior annular cross-section of shaft 28 to define each lumen 30.

Sheath 32 is a flexible tubular member that extends longitudinally a distance D from knob 20 along centerline 44 to terminal end 32A and circumscribes shaft 28. Sheath 32 has an annular cross-section taken normal to centerline 44. Sheath 32 can extend up to the entire length of shaft 28 such that distance D equals length L. However, as distance D increases, the flexibility of the shaft-sheath or shaft-sheath-inner-shaft combination decreases. Accordingly, in most instances, distance D of sheath 32 is shorter than length L of catheter 10, overlapping only a portion of shaft 28.

Implant 14 is a device or sensor that can attach to a patient via a catheter (e.g. catheter 10). Implantable devices can be anchors or adapters containing a port for receiving a subsequently implanted device. Each type of implant 14 includes a ferromagnetic component to facilitate retention to catheter 10 via magnet 36.

Exemplary features of implant 14 are illustrated by FIG. 1B and FIG. 1C. As shown, implant 14 includes body 46 and, in some embodiments, helical wire 48. At least one of body 46 and helical wire 48, or a portion of body 46 or helical wire 48 contains ferromagnetic metal to facilitate retention by magnetic release mechanism 12.

Exterior peripheral surface 46A of body 46 can be axisymmetric about centerline 44, shown for reference in FIG. 1B and FIG. 1C. When paired with a complementary interior surface of socket 40, axisymmetric exterior peripheral surface 46A rotationally uncouples implant 14 from shaft 28. Rotational restraint of implant 14 about centerline 44 can be provided by magnetic release mechanism 12 and facilitates magnetic release mechanism 12 as a device for limiting torsional input from catheter 10 into the patient.

As an anchor, body 46 can include bore 50, which extends entirely through body 46. Bore 50 may be used to connect multiple implants 14 within the patient. For example, bores 50 of adjacent implants 14 can be connected by threading a suture through each bore 50 to connect adjacent implants 14 or connect implant 14 to a different implantable device. In other instances, body 46 may form port 52 to receive a different implant or device to ease attachment and detachment of that implant or device. An interior peripheral surface 52A of port 52 conforms to an exterior surface of the mating implant or device and a depth of port 52 accommodates at least a portion of the mating implant or device. Moreover, implant 14 can be a sensor such that body 46 is a pressure sensor body or a temperature sensor body. In any of the foregoing embodiments, helical wire 48 may extend from an end of body 46 opposite an end that abuts spacer wall 38 of shaft 28. Rotating body 46 about centerline 44 with magnet 36 rotates helical wire 48, boring wire 48 into tissue of the patient and securing implant 14 to the patient at the attachment site.

While multiple features of implant 14 are depicted simultaneously by FIG. 1B and FIG. 1C, implant 14 can include any one of the foregoing features, a subset of the foregoing features, or all of the foregoing features as required. While FIG. 1B and FIG. 1C depict a single bore 50 and/or port 52, some embodiments of implant 14 may include multiple bores 50, ports 52, or multiple bores 50 and multiple ports 52.

FIG. 1D is a cross-sectional view showing proximal end 10A of catheter 10 in greater detail. Handle 16 includes passage 54 extending from distal end 16A to proximal end 16B of handle 16 and bound by interior surface 56. Exterior surface 58 of handle 16 can be contoured to facilitate hand-manipulation by a practitioner.

Knob 20 attaches to handle 16 at distal end 16A such that handle 16 axially restrains knob 20 while knob 20 rotates freely about centerline 44 with respect to handle 16. As shown in FIG. 1D, knob 20 can include flange 60 extending radially inward into passage 54. Flange 60 is axisymmetric about centerline 44 and forms circumferentially-extending groove 62A positioned opposite and axially coinciding with groove 62B, which protrudes into handle 16 from interior surface 56. Corresponding grooves 62A and 62B restrain capture ring 64 and thereby axially restrain knob 20 with respect to handle 16. Since capture ring 64 is devoid of rotational restraints (e.g., tabs, protrusions, etc.), knob 20 freely rotates about centerline 44 with respect to handle 16. In other embodiments, capture ring 64 and associated features can be replaced with a bushing or a bearing that is installed radially between exterior surface 66 of flange 60 and interior surface 56 of handle 16. In these embodiments, a location fit or interference fit between exterior surface 66 of flange 60 and interior surface 56 of handle 16 axially restrains knob 20 with respect to handle 16. In other examples, a key or snap ring retained within corresponding keyways or snap ring grooves axially restrain knob 20 with respect to handle 16.

Knob 20 includes flange 68 extending in a distal direction opposite flange 60. Similar to flange 60, flange 68 is axisymmetric about centerline 44. Additionally, exterior surface 70 of flange 68 receives and axially restrains shaft 28. Furthermore, installing bushing 72 between exterior surface 70 of flange 68 and an interior surface of shaft 28 permits knob 20 to rotate about centerline 44 with respect to shaft 28.

Handle 16, knob 20, and shaft 28 circumscribe inner shaft 34 and terminal member 24 used to operate magnetic release mechanism 12 at proximal end 10A. Inner shaft 34 is a flexible member with an annular, circular, elliptical, or ovular cross section that extends from handle 16, within lumen 30, to magnetic release mechanism 12.

Shaft 28, sheath 32, and inner shaft 34 are non-magnetic. Acceptable non-magnetic materials for shaft 28, sheath 32, and inner shaft 34 retain sufficient longitudinal stiffness to avoid buckling under longitudinal forces imposed on catheter 10 by a practitioner when inserted into the patient. While shaft 28, sheath 32, and inner shaft 34 can be constructed from a variety of materials, acceptable thermoplastic materials may include polyamide (PA), fluorinated ethylene-propylene (FEP), tetrafluoroethylene (TFE), hexafluoropropylene (HFP), fluoropolymer, polyester-polyether copolymer, polyether ether-ketone (PEEK), high density polyethylene (HDPE), low density polyethylene (LDPE), polyester, polyurethane, and polypropylene. Materials for shaft 28, sheath 32, and inner shaft 34 can be the same or different from each other. Moreover, interior surfaces, exterior surfaces, or both interior and exterior surfaces of shaft 28, sheath 32, and/or inner shaft 34 can be coated with a hydrophilic lubricant and/or an antimicrobial among other possible medical coatings.

Terminal member 24 is a rod or tube connected to inner shaft 34 at proximal end 10A of catheter 10 that is housed entirely within handle 16, knob 20, and shaft 28. External threads 74A of terminal member 24 engage internal threads 74B of knob 20. Additionally, terminal member 24 is rotationally restrained relative to handle 16 at coupling 26. For instance, terminal member 24 can be rotationally coupled via key 75 housed within corresponding keyways 76A and 76B of the terminal member 24 and handle 16. In other embodiments, terminal member 24 may include one or more radially-extending tabs or protrusions that interface with corresponding tabs or protrusions extending radially inward from handle 16. In each case, terminal member 24 translates freely in an axial or longitudinal direction along centerline 44 while rotationally restrained about centerline 44 relative to handle 16.

Catheter 10 can include end cap 18 enclosing proximal end 16B of handle 16. In such embodiments, end cap 18 can include external threads 78A that engage corresponding internal threads 78B formed by interior surface 56 of handle 16. In other embodiments, end cap 18 can have a tubular projection that presses into passage 54 of handle 16 that thereby forms a location fit or interference fit between interior surface 56 of handle 16 and an exterior surface of the end cap protrusion. End cap 18 and terminal member 24 compresses spring 22 retained therebetween to impose preload on terminal member 24 and thereby preload magnetic release mechanism 12 via inner shaft 34. The preload on magnetic release mechanism 12 imposed by spring 22 prevents mechanism 12 from disengaging inadvertently from the manipulation of catheter 10 during the catheterization procedure.

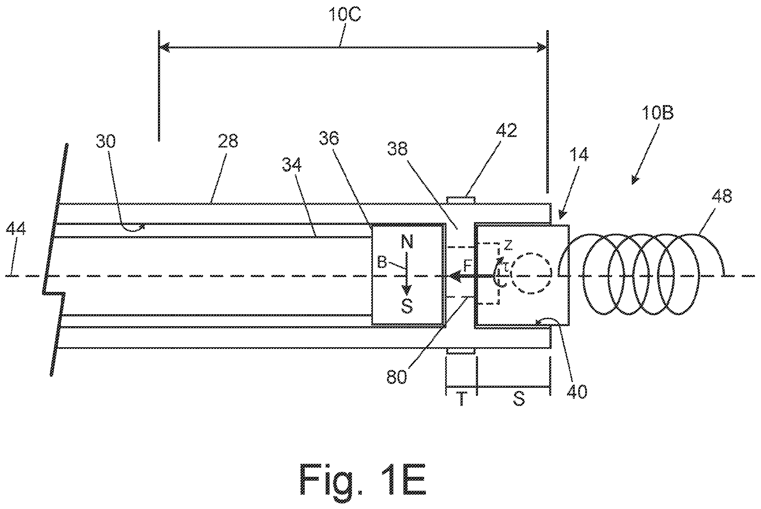

FIG. 1E is a cross-sectional view showing distal end 10B of catheter 10 in greater detail. At distal end 10B of catheter 10, spacer wall 38 encloses lumen 30. In some instances, spacer wall 38 encloses lumen 30 at distal end 10B of catheter 10. In other embodiments and as shown in FIG. 1E, offsetting spacer wall 38 from distal end 10B of catheter 10 forms socket 40 open to distal end 10B of catheter 10. The longitudinal spacing S of spacer wall 38 measured from the distal end of shaft 28 can accommodate at least a fractional length of implant 14 and, in other embodiments, can accommodate up to the entire length of implant 14 within socket 40. The position of spacer wall 38 locates magnet 36 within distal region 10C of catheter 10. In embodiments useful for understanding the presently claimed invention, the longitudinal extent of distal region 10C can be within five diameters of shaft 28, or lumen 30, from distal end 10B. In other embodiments useful for understanding the presently claimed invention, the distal region may encompass more or less than five diameters of shaft 28 or lumen 30 from distal end 10B.

Spacer wall 38 is formed from a non-magnetic material. In some embodiments, spacer wall 38 is injected molded using the same material as shaft 28 and subsequently fused to shaft 28 with heat. In other embodiments, spacer wall 38 may be integrally formed at the same time as shaft 28.

Magnet 36 can be a permanent magnet that attaches to a distal end of inner shaft 34. Both inner shaft 34 and magnet 36 are housed within lumen 30. In an engaged state, magnet 36 abuts spacer wall 38 such that magnetic flux density B of magnet 36 intersects implant 14 to produce an induced magnetic field within implant 14 that aligns with the magnetic field orientation of magnet 36. The magnetic force F produced between the magnetic dipole of magnet 36 and the induced magnetic dipole of implant 14 acts normal to spacer wall 38. Magnetic flux density B of magnet 36 at a location within its magnetic field can be increased or decreased based on material size, and or shape of magnet 36. Increasing or decreasing magnetic flux density B intersecting implant 14 increases or decreases magnetic force F, respectively. Furthermore, increasing thickness T of spacer wall 38 decreases magnetic flux density B intersecting implant 14 while decreasing thickness T of spacer wall 38 increases magnetic flux density B intersecting implant 14. Additionally, one or more holes 80 can extend through spacer wall 38. With each additional hole 80, or as an area of hole 80 increases, magnetic flux density B intersecting implant 14 increases. Accordingly, varying magnetic flux density B by specifying a material, size, and shape of magnet 36, selecting thickness T of spacer wall 38, adding one or more holes 80, or any combination of the foregoing features modifies the longitudinal retention force due to magnetic force F.

The dipole of magnet 36 can be orientated perpendicular to centerline 44 or the longitudinal direction of shaft 28. In this situation, the dipole of the magnetic field induced within implant 14 aligns with the dipole of magnet 36. When rotation of magnet 36 about centerline 44 produces an angular deviation between the dipoles, a correcting torque is imposed on implant 14 until the dipoles of magnet 36 and implant 14 realign. Accordingly, the magnetic field of magnet 36 can restrain implant 14 in a rotational direction about centerline 44.

Magnet 36 couples rotationally to handle 16 such that torsional input applied to handle 16 about centerline 44 rotates magnet 36 and indirectly rotates implant 14 at distal end 10B or within socket 40 of catheter 10. By adjusting the magnetic flux density B intersecting implant 14, magnet 36 can limit the reaction force imposed on implant 14 by the patient's tissue. In such embodiments, excessive torsional input at the handle causes implant 14 to overcome the magnetic correcting torque imposed by magnet 36, rotationally decoupling implant 14 from magnet 36 to prevent or limit tissue damage.

Accordingly, magnetic release mechanism 12 can be configured to be a torque limiter. In some instances, the torsional restraint of implant 14 within socket 40 can be selected such that a reaction force at the interface between implant 14 and the patient is between five newtons (5N) and thirty newtons (30N). In other embodiments, the reaction force at the interface between implant 14 and the patient can be between five newtons (5N) and twenty newtons (20N) or between five newtons (5N) and ten newtons (10N).

Distal end 10B of catheter 10 can be equipped with marker strip 42. As shown in FIG. 1E, marker strip 42 extends circumferentially about an exterior surface of shaft 28 at a location aligned with spacer wall 38. In some cases, marker strip 42 can be placed at distal end 10B of catheter 10 while in other instances, marker strip 42 can be spaced from distal end 10B of catheter 10 at a location that does not coincide with spacer wall 38. Marker strip 42 is formed by portions of shaft 28, or another component of catheter 10, that are impregnated with materials detectable by fluoroscopy. In this case, marker strip 42 can be a segment of shaft 28 impregnated with tungsten or platinum, materials that are readily detectable by fluoroscopy.

Magnetic release mechanism 12 selectively engages or disengages based on a position of magnet 36 relative to spacer wall 38. In the engaged state, magnet 36 abuts spacer wall 38. Implant 14 is retained axially by magnetic force F, retained laterally by socket 40, and in some instances, rotationally restrained about centerline 44 by a magnetic correcting torque τ. Rotational manipulation of handle 16 about centerline 44 rotates terminal member 24 via coupling 26 and thereby rotates inner shaft 34 and magnet 36 within lumen 30. As magnet 36 rotates within lumen 30, implant 14 rotates as an induced dipole within ferromagnetic components of implant 14 realign with the dipole of magnet 36. As such, implant 14 of catheter 10 may apply torque to implant 14, anchoring implant 14 to an attachment site within a patient while excess torque applied at handle 16 uncouples implant 14 from catheter 10 in a rotational direction about centerline 44.

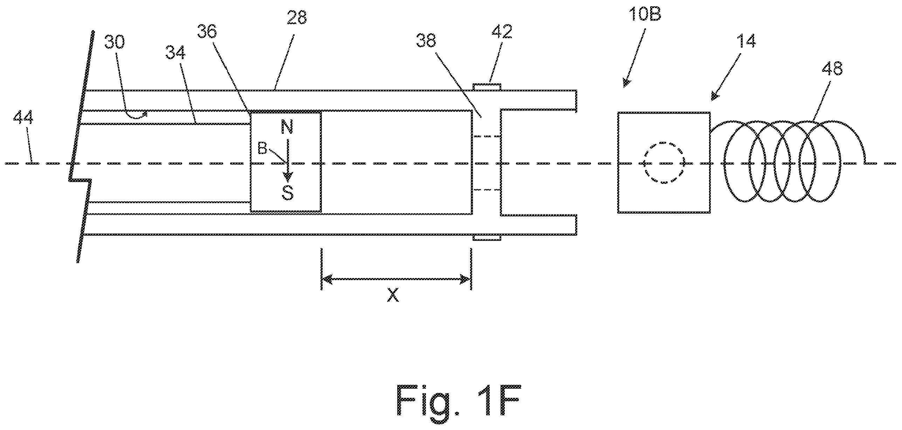

Once implant 14 attaches to the patient, magnetic release mechanism 12 axially decouples catheter 10 from implant 14 by rotating knob 20. As knob 20 rotates about centerline 44, terminal member 24 translates axially in a proximal direction within handle 16 while coupling 26 rotationally restrains terminal member 24 with respect to handle 16. Translation of terminal member 24 corresponds to displacement of inner shaft 34 and magnet 36 in a proximal direction away from spacer wall 38. Once magnet 36 displaces beyond the effective range of magnet 36 as shown in FIG. 1F, implant 14 uncouples from catheter 10. While displacement distance X may vary based on magnetic flux density B, displacement distance X ranges from 3 millimeters to 15 millimeters in some embodiments. In other embodiments, displacement distance X ranges from 3 millimeters to 10 millimeters or between 3 millimeters to 5 millimeters.

Accordingly, magnetic release mechanism 12 of catheter 10 reduces the number of moving parts within distal region 10C of catheter 10 as compared to an analogous mechanical release mechanism (e.g., grips, clamps, and other holding attachments and adapters). By contrast, magnetic release mechanism 12 includes a single movable assembly within distal region 10C formed by inner shaft 34 and magnet 36, which translates longitudinally along centerline 44 and rotates about centerline 44 in response to knob 20 and handle 16 manipulation at proximal end 10A of catheter 10. Furthermore, reducing complexity of the release mechanism reduces catheter cross-section normal to centerline 44. These features facilitate attachment of one or more implants 14 within a patient using a minimally invasive catheterization procedure rather than an invasive surgical procedure.

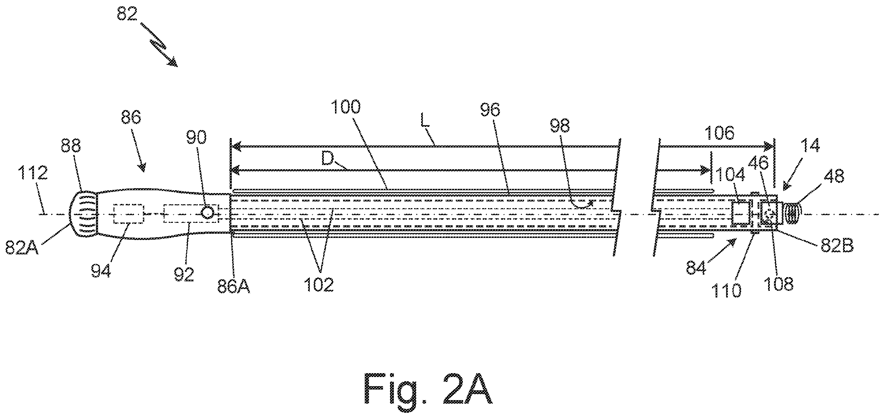

FIG. 2A depicts catheter 82 equipped with magnetic release mechanism 84. At proximal end 82A, catheter 82 includes handle 86, end cap 88, selector 90, drive circuit 92, and power supply 94. Extending from proximal end 82A towards distal end 82B, catheter 82 includes shaft 96, lumen 98, sheath 100, and leads 102. At distal end 82B, catheter 82 includes magnet 104, spacer wall 106, socket 108, marker strip 110, and implant 14. However, magnetic release mechanism 84 is formed by magnet 104, drive circuit 92, power supply 94, leads 102, and selector 90 and does not include inner shaft 34, terminal member 24, and knob 20 utilized by magnetic release mechanism 12.

Length L of catheter 82 is measured longitudinally along centerline 112 from distal end 86A of handle 86 to distal end 82B of catheter 82 and corresponds to a longitudinal direction of catheter 82 and components of catheter 82. Catheter length L of catheter 82 can be adapted to the intended insertion site and attachment site in the same manner as catheter 10. A typical length of catheter 82 will be between 60cm and 110cm but can be longer or shorter as required for the procedure or permitted by the component materials of catheter 82.

Shaft 96 is a flexible tubular member like shaft 28 of catheter 10. However, shaft 96 extends from distal end 86A of handle 86 rather than knob 20. Shaft 96 forms one or more lumens 98, which extend from a proximal end to a distal end of shaft 96, or from a proximal end of shaft 96 to spacer wall 106. Shaft 96 has an annular cross-section take normal to centerline 112 that may or may not include partition walls for additional lumens 98.

Sheath 100 of catheter 82 is similarly constructed to sheath 32 of catheter 10. As such, sheath 100 has an annular cross-section take normal to centerline 112 and extends a distance D from distal end 86A of handle 86, rather than knob 20, towards distal end 82B of catheter 82 to a terminal end. While distance D of sheath 100 can be up to the length L of catheter 82, distance D of sheath 100 is shorter than length L of catheter 82, overlapping a portion of shaft 96.

Implant 14 is a device or sensor that can attach to a patient via catheter (e.g., catheter 82) and can be identical to implants 14 used with catheter 10. As such, exemplary features of implant 14 include body 46, helical wire 48, bore 50, port 52 as depicted in FIG. 1B and FIG. 1C.

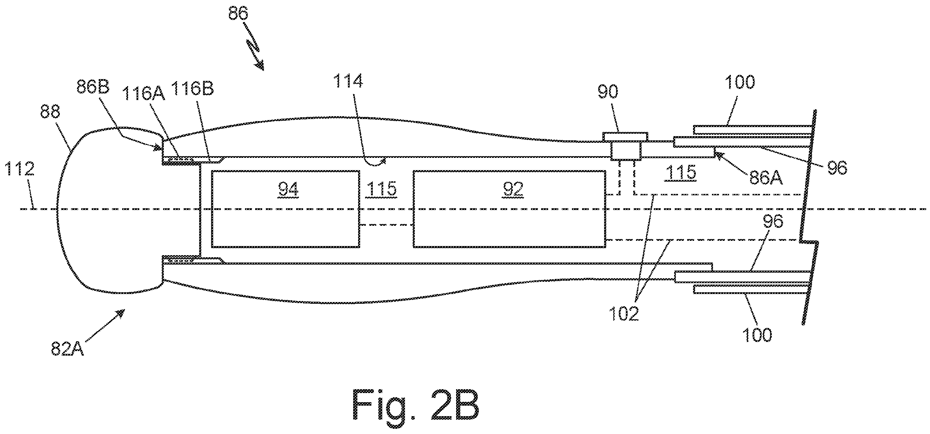

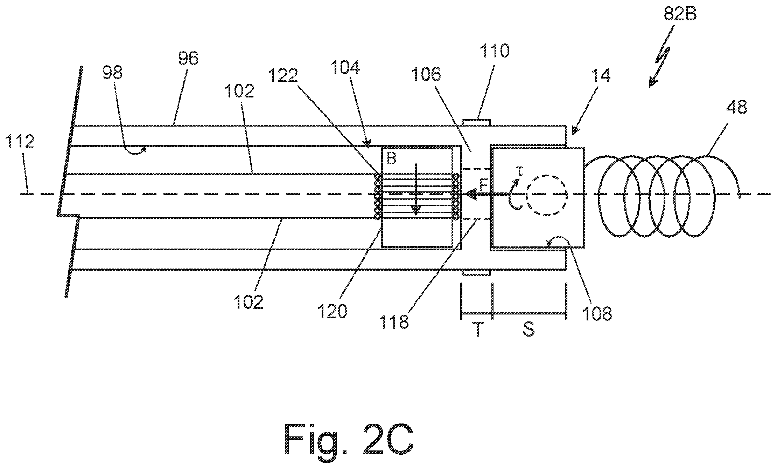

Magnet 104 is an electromagnet rotationally and longitudinally coupled to shaft 96 that abuts spacer wall 106 within lumen 98 in contrast to magnet 32, which translates within lumen 30 via inner shaft 34, terminal member 24, coupling 26, and knob 20. Drive circuit 92 and power supply 94 couple electrically to magnet 104 via leads 102. While leads 102 are shown separately in FIG. 2A, FIG. 2B, and FIG. 2C, leads 102 can form a twisted pair, which extend within lumen from handle 86 to magnet 104. Without inner shaft 34, terminal member 24 and associated features of handle 16, the cross-sectional profile of shaft 96 taken normal to a centerline of catheter 82 can be reduced relative to shaft 28 of catheter 10.

FIG. 2B is a cross-sectional view of proximal end 82A of catheter 82. Like handle 16, interior surface 114 of handle 86 defines passage 115 extending longitudinally through handle 86 from distal end 86A to proximal end 86B. Housed within handle 86, drive circuit 92 operates to regulate electrical power delivered to magnet 104 disposed at distal end 82B of catheter 82. Drive circuit 92 outputs electrical power via leads 102, which extend within lumen 98 from drive circuit 92 to magnet 104. For instance, drive circuit 92 may deliver a constant or variable current through leads 102 at a constant voltage to magnet 104.

Electrical power can be provided by power supply 94, which may be housed within handle 86 and directly supplying power to drive circuit 92. For example, power supply 94 can be a battery contained within handle 86 of catheter 82. Alternatively, power supply 94 can be external to catheter 82 and electrically connected to drive circuit 92 through a cable penetrating or attached to leads 102 through handle 86. For example, power supply 94 can be a constant voltage supply that coverts electrical power from an AC source to a regulated current output at a constant voltage.

Selector 90 mounts to handle 86 and electrically interfaces with one or more of drive circuit 92, power supply 94, and leads 102. In some embodiments, selector 90 can be electrically connected to drive circuit 92 and operable to vary the current supplied to magnet 104. For example, selector 90 can be a dial operable to vary the resistance of an analog or digital potentiometer of drive circuit 92 that thereby varies the output current to leads 102. In other embodiments, selector 90 is a switch operable to open and close the electrical circuit to magnet 104 such as by interrupting one or both of leads 102 or interrupting a connection between power supply 94 and drive circuit 92.

Shaft 96 attaches to distal end 86A of handle 86 such that shaft 96 is fully restrained with respect to handle 86. For example, shaft 96 can be attached to handle 86 using a ferrule attachment whereby a ferrule nut traps a proximal end of shaft 96 against a corresponding taper portion formed at distal end 86A of handle 86. In other instances, shaft 96 can be press fit onto a tubular sleeve or barbed connector protruding from distal end 86A of handle 86.

Catheter 82 can include end cap 88 to enclose proximal end 86B of handle 86. In such embodiments, end cap 88 can include external threads 116A that engage corresponding internal threads 116B formed by interior surface 114 of handle 86. In other embodiments, end cap 88 can have a tubular projection that presses into passage 115 of handle 86 that thereby forms a location fit or interference fit between interior surface 114 of handle 86 and an exterior surface of the end cap protrusion. In other embodiments, handle 86 can be enclosed at proximal end 86B such that passage 115 does not extend entirely through handle 86. Such close-ended handle 86 designs do not require end cap 88.

FIG. 2C depicts a cross-sectional view of distal end 82B of catheter 82. Similar to catheter 10, spacer wall 106 encloses lumen 98 at distal end 82B. In other embodiments and as shown in FIG. 2C, offsetting or spacing spacer wall 106 from distal end 82B of catheter 82 forms socket 108 open to at distal end 82B of catheter 82. The longitudinal distance S of spacer wall 106 measured from the distal end 82B can be selected to partially accommodate a length of implant 14 and, in other embodiments, can accommodate up to the entire length of implant 14 within socket 108.

Spacer wall 106 is similar to spacer wall 38 of catheter 10. As such, spacer wall 106 is formed from a non-magnetic material, which can be manufactured or formed in the same manner as spacer wall 38. Additionally, spacer wall 106 can include one or more through holes 118 that can be arranged and function in the same way has through holes 80 of catheter 10.

Magnet 104 includes core 120 and winding 122 circumscribing core 120. The magnetic flux density B intersecting implant 14 is proportional to the direct current magnitude supplied by drive circuit 92. Increasing direct current magnitude corresponds to increased magnetic flux density B, and decreasing direct current magnitude corresponds to decreasing magnetic flux density B intersecting implant 14. Additionally, the magnetic flux density B of magnet 104 increases as turns of winding 122 are added to magnet 104 and decreases with fewer turns of winding 122. Additionally, magnetic flux density B can be increased or decreased as a function of thickness T of spacer wall 106 in the same manner as spacer wall 38.

In operation, power supply 94 provides electrical power to drive circuit 92, which in turns delivers direct current at constant voltage to magnet 104. When drive circuit 92 supplies direct current, magnet 104 produces a magnetic field, which in turn, restrains implant 14 rotationally about centerline 112 and longitudinally along centerline 112 within socket 108, or in other embodiments, at a distal end of catheter 82. Once practitioners manufacture distal end 82B of catheter 82 to a desired attachment location, rotation of handle 86 about centerline 112 rotates shaft 96. Since magnet 104 is rotationally coupled to shaft 96, rotation of shaft 96 translates to implant 14 via the magnetic field of magnet 104 in the same manner as catheter 10. Accordingly, rotation of shaft 96 can be used to attach implants 14 equipped within rotational attachments (e.g., helical wire 48) to a patient. Disengagement of magnetic release mechanism occurs when a state of selector 90 disrupts direct current output from drive circuit 92. Without direct current, magnetic flux density B of magnet 104 dissipates, rotationally and longitudinally uncoupling implant 14 form catheter 82.

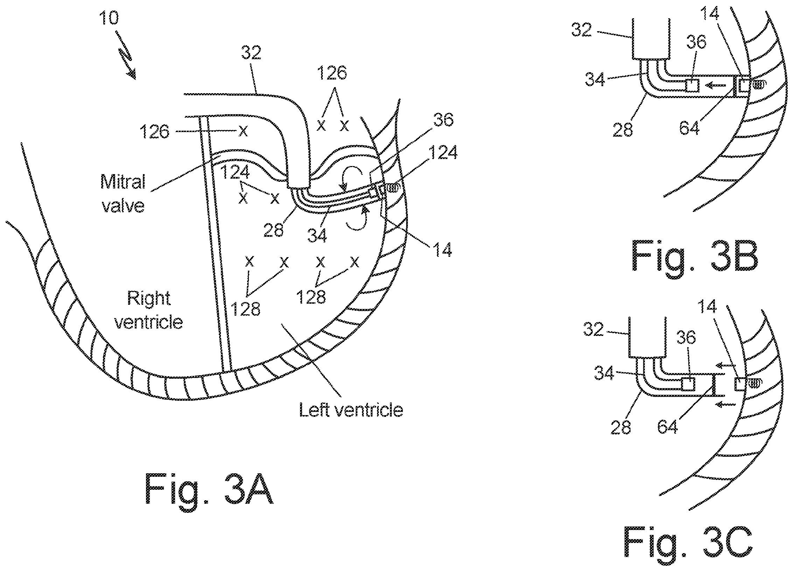

FIG. 3A is a schematic showing several exemplary attachment sites for implant 14. While the following examples refer to catheter 10, catheter 82 may be used in place of or in addition to catheter 10 for other procedures. As shown in FIG. 3A, catheter 10 entered into the patient's left ventricle through the mitral valve via the left atrium and the pulmonary vein. Rotating catheter 10 about centerline 44 via handle 16 causes magnet 36 to rotate within lumen 30 that thereby causes implant 14 to rotate. As depicted, implant 14 includes a helical anchor that bores into the patient's heart tissue in a subannular region interior of the mitral valve as indicated at sites 124. Other exemplary attachment sites within the patient's heart include super annular locations of the patient's left atrium tissue and mid ventricular locations within the patient's left ventricle, each indicative in FIG. 3A at 126 and 128, respectively.

In operation, implant 14 attaches to the patient's heart tissue by manipulating handle 16 of catheter 10 as shown in FIG. 3A. After disengaging magnetic release mechanism 12, catheter 10 maintains position of implant 14 as shown in FIG. 3B. Subsequently, catheter 10 withdraws from implant 14 as shown in FIG. 3C.

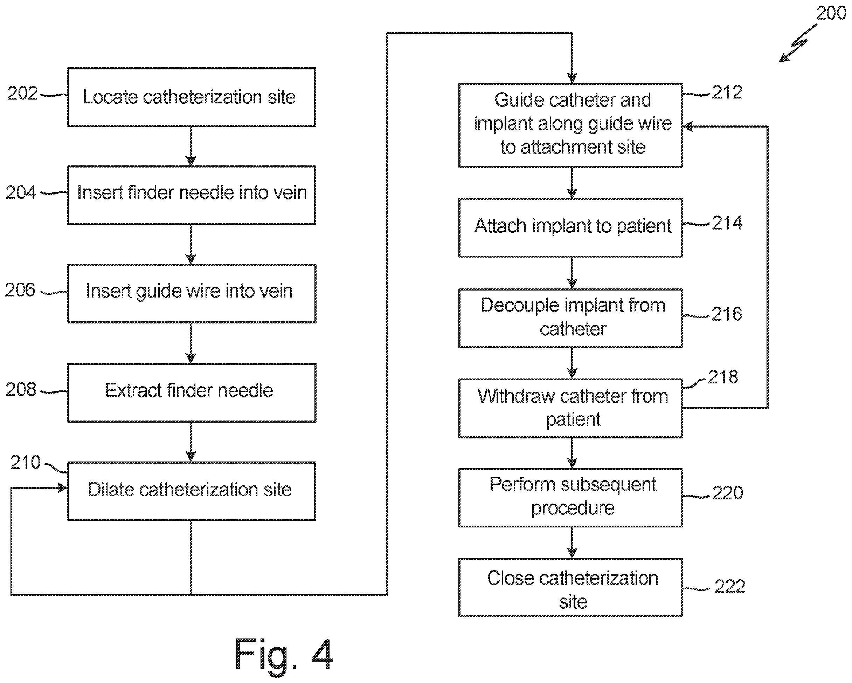

FIG. 4 is a flowchart describing a procedure for attaching implant using catheter 10 or catheter 82, which have been sterilized prior to use. Method 200 includes at least steps 212, 216, and 218. Additionally, method 200 can include one or more of steps 202, 204, 206, 208, 210, 214, 220 and 222 as described below. The sequence depicted is for illustrative purposes only and is not meant to limit method 200 in any way as it is understood that the portions of the method can proceed in a different logical order, additional or intervening portions can be included, or described portions of the method can be divided into multiple portions, or described portions of the method can be omitted without detracting from the described method below.

In steps 202, 204, 206, 208, 210, and 212, a practitioner prepares a patient for catheterization. In step 202, a practitioner locates a patent's vein using ultrasound inspection techniques. After locating a patient's vein, the practitioner inserts a finder needle into the patient at the vein site in step 204. Equipped with a syringe, the practitioner draws negative pressure on the finder needle simultaneously with inserting the finder needle towards a patient's vein. Once the finder needle intercepts the patient's vein, the practitioner inserts a guide wire through the finder needle into the patient's vein in step 206. In step 208, the practitioner extracts the finder needle, leaving the guide wire inserted within the vein. Subsequently in step 210, the practitioner may guide a dilator onto the guide wire, make a small incision in the patient at the vein site to accommodate the dilator, insert the dilator, and expand the patient's soft tissue to facilitate insertion of the catheter. Step 210 can be repeated with larger dilators until the vein site has been enlarged to accommodate one of catheters 10 and 82.

At this stage, the practitioner guides catheter 10 (or catheter 82), which is equipped with implant 14, along the guide wire into the patient's vein in step 212. During the catheterization procedure, the practitioner may guide catheter 10 (or catheter 82) by advancing, retracting, and/or twisting catheter 10 (or catheter 82) via manipulation of handle 16 (or handle 86). After guiding catheter 10 (or catheter 82) to the attachment side, the practitioner may rotate handle 16 (or handle 86) about centerline 44 (or centerline 112) to thereby rotate implant 14 at the attachment site. For implants 14 equipped with rotational mechanical couplers (e.g., a helical anchor), the practitioner may engage the implant's mechanical coupler by rotating handle 16 or (handle 86) about its centerline, which transfers rotation to implant 14 via permanent magnet 36 or electromagnet 104 in step 214. In step 216, the practitioner disengages magnetic release mechanism 12 (or magnetic release mechanism 84). For catheter 10, magnetic release mechanism 12 disengages by rotating knob 20 and thereby retracting magnet 36. Where catheter 82 is used, magnetic release mechanism 84 disengages by disrupting direct current to magnet 104 via selector 90. In step 218, the practitioner withdraws catheter 10 (or catheter 82) from the patient.

Some procedures require multiple implants 14. Accordingly, steps 212, 214, 216 and 218 can be repeated, as required, to attach subsequent implants 14. Implants 14 can be configured identically to previous implants, or implant 14 can have a different configuration than prior implants 14. Additionally, subsequent implants 14 can be implanted at or near the same attachment site or a different attachment site as required by the procedure.

After withdrawing catheter 10 (or catheter 82) in step 218, the practitioner may perform a different catheterization procedure through the same catheterization site for monitoring the patient during subsequent procedures in step 220. Alternatively, the practitioner may withdraw any remaining sheaths and/or dilator before closing the patient's catheterization site in step 222.

While the invention has been described with reference to an exemplary embodiment(s), it will be understood by those skilled in the art that various changes may be made without departing from the scope of the invention. In addition, many modifications may be made to adapt a particular situation or material to the teachings of the invention without departing from the essential scope thereof. Therefore, it is intended that the invention is not limited to the particular embodiment(s) disclosed, but that the invention will include all embodiments falling within the scope of the appended claims.

## Claims

1. A catheter (10) for delivering an implant (14) to a patient, the catheter comprising:
a shaft (28) defining a lumen (30) extending from a proximal end (10A) to a distal end (10B) of the shaft, the shaft comprising:
a spacer wall (38) enclosing the lumen within a distal region (10C) of the shaft, wherein the distal region extends from the distal end towards the proximal end a distance equal to five diameters of the shaft; and
a magnetic release mechanism (12) comprising:
a magnet (36) disposed within the lumen, wherein the magnet is disposed within the distal region of the lumen between the spacer wall and the proximal end.

2. The catheter of claim 1, further comprising:
a marker strip (42) circumscribing an exterior of the lumen and aligning with the spacer wall.

3. The catheter of claim 1 or claim 2, wherein the spacer wall is spaced from the distal end to form a socket (40) open to the distal end of the shaft.

4. The catheter of claim 3, further comprising:
an implant (14) at least partially received within the socket.

5. The catheter of claim 4, wherein the implant includes a helical anchor wire (48) extending from an outward surface of the implant opposite an inward surface of the implant abutting the spacer wall.

6. The catheter of claim 4, wherein the implant is rotationally restrained within the socket by a magnetic field of the magnet.

7. The catheter of claim 6, wherein a magnetic axis of the magnet is perpendicular to a centerline (44) of the shaft, and wherein a maximum magnetic torque imposed on the implant imposes no more than a threshold force onto the implant, the threshold force between 5 newtons and 30 newtons.

8. The catheter of any preceding claim, wherein the magnetic release mechanism further comprises:
an inner shaft (34) joined to the magnet and extending within the lumen from the magnet to the proximal end, wherein the magnet is a permanent magnet.

9. The catheter of claim 8, further comprising:
a handle (16); and
a bushing (72) affixed to the handle and the shaft at the proximal end, wherein the handle restrains linear displacement of the bushing and the shaft with respect to the handle, and wherein the shaft is rotatable with respect to the handle about a centerline of the handle.

10. The catheter of claim 9, further comprising:
a knob (20) joined to the handle; and
a terminal member (24) affixed to the inner shaft and coinciding with the handle, wherein external threads (74A) of the terminal member engage internal threads (74B) of the knob such that rotation of the knob displaces the magnet within the lumen with respect to the spacer wall.

11. The catheter of claim 10, further comprising:
an end cap (18) joined to the handle opposite the knob; and
a spring (22) compressed within the handle between the end cap and the terminal member.

12. The catheter of any preceding claim, further comprising:
a sheath (32) encircling the shaft and extending from the proximal end to a terminal end (32A) spaced from the distal end of the shaft, wherein the shaft protrudes from the sheath at the distal end.

13. The catheter of claim 12, when claim 12 is dependent upon any one of claims 1-6, further comprising:
a handle (16) affixed to the shaft at the proximal end, wherein the handle restrains linear and rotational displacement of the shaft with respect to the handle.

14. The catheter of claim 13, wherein the magnet is an electromagnet, the electromagnet comprising:
a core (120);
a winding (122) encircling the core; and
leads (102) extending from the windings to the handle electrically connecting the electromagnet to a switch mounted to the handle,
optionally wherein the magnetic release mechanism further comprises:
a power supply (94) electrically connected to the switch, wherein the switch includes an open state that electrically disconnects the power supply from the electromagnet and a closed state that electrically connects the power supply to the electromagnet.

15. The catheter of any preceding claim, wherein the catheter is sterilized.

## Patentansprüche

1. Katheter (10) zum Zuführen eines Implantats (14) in einen Patienten, wobei der Katheter umfasst:
einen Schaft (28), der ein Lumen (30) definiert, das sich von einem proximalen Ende (10A) zu einem distalen Ende (10B) des Schafts erstreckt, wobei der Schaft umfasst:
eine Abstandswand (38), die das Lumen innerhalb eines distalen Bereichs (10C) des Schafts umgibt, wobei sich der distale Bereich von dem distalen Ende in Richtung des proximalen Endes über eine Distanz erstreckt, die fünfmal dem Durchmesser des Schafts entspricht; und
einen magnetischen Freigabemechanismus (12), der umfasst:
einen Magneten (36), der innerhalb des Lumens angeordnet ist, wobei der Magnet innerhalb des distalen Bereichs des Lumens zwischen der Abstandswand und dem proximalen Ende angeordnet ist.

2. Katheter nach Anspruch 1, ferner umfassend:
einen Markierstreifen (42), der eine Außenseite des Lumens umschreibt und auf die Abstandswand ausgerichtet ist.

3. Katheter nach Anspruch 1 oder Anspruch 2, wobei die Abstandswand von dem distalen Ende beabstandet ist, um eine Aufnahme (40) zu bilden, die zum distalen Ende des Schafts hin offen ist.

4. Katheter nach Anspruch 3, ferner umfassend:
ein Implantat (14), das mindestens teilweise in der Aufnahme aufgenommen ist.

5. Katheter nach Anspruch 4, wobei das Implantat einen spiralförmigen Verankerungsdraht (48) aufweist, der sich von einer Außenseite des Implantats gegenüber einer Innenseite des an der Abstandswand anliegenden Implantats erstreckt.

6. Katheter nach Anspruch 4, wobei das Implantat durch ein Magnetfeld des Magneten drehbar in der Aufnahme gehalten wird.

7. Katheter nach Anspruch 6, wobei eine Magnetachse des Magneten senkrecht zu einer Mittellinie (44) des Schafts verläuft, und wobei ein dem Implantat aufgezwungenes maximales magnetisches Drehmoment dem Implantat nicht mehr als eine Schwellenkraft aufzwingt, wobei die Schwellenkraft zwischen 5 Newton und 30 Newton beträgt.

8. Katheter nach einem vorhergehenden Anspruch, wobei der magnetische Freigabemechanismus ferner umfasst:
einen Innenschaft (34), der mit dem Magneten verbunden ist und sich innerhalb des Lumens vom Magneten zum proximalen Ende erstreckt, wobei der Magnet ein Permanentmagnet ist.

9. Katheter nach Anspruch 8, ferner umfassend:
einen Griff (16); und
ein Lager (72), das an dem Griff und dem Schaft an dem proximalen Ende angebracht ist, wobei der Griff die lineare Verschiebung des Lagers und des Schafts mit Bezug auf den Griff zurückhält; und wobei der Schaft mit Bezug auf den Griff um eine Mittellinie des Griffs drehbar ist.

10. Katheter nach Anspruch 9, ferner umfassend:
einen Knopf (20), der mit dem Griff verbunden ist; und
ein terminales Element (24), das an dem Innenschaft angebracht ist und mit dem Griff zusammenfällt, wobei Außengewinde (74A) des terminalen Elements Innengewinde (74B) des Knopfes in Eingriff nehmen, so dass die Drehung des Knopfes den Magneten innerhalb des Lumens mit Bezug auf die Abstandswand verschiebt.

11. Katheter nach Anspruch 10, ferner umfassend:
eine Endkappe (18), die gegenüber des Knopfes mit dem Griff verbunden ist; und
eine Feder (22), die in dem Griff zwischen der Endkappe und dem terminalen Element komprimiert ist.

12. Katheter nach einem vorhergehenden Anspruch, ferner umfassend:
eine Hülle (32), die den Schaft umgibt und sich von dem proximalen Ende zu einem terminalen Ende (32A) erstreckt, das von dem distalen Ende des Schafts beabstandet ist, wobei der Schaft von der Hülle an dem distalen Ende vorspringt.

13. Katheter nach Anspruch 12, wenn Anspruch 12 von einem der Ansprüche 1-6 abhängt, ferner umfassend:
einen Griff (16), der an dem proximalen Ende an dem Schaft angebracht ist, wobei der Griff die lineare Verschiebung und die Drehverschiebung des Schafts mit Bezug auf den Griff zurückhält.

14. Katheter nach Anspruch 13, wobei der Magnet ein Elektromagnet ist, wobei der Elektromagnet umfasst:
einen Kern (120);
eine Wicklung (122), die den Kern umgibt; und
Leitungen (102), die sich von den Wicklungen zu dem Griff erstrecken, die den Elektromagneten elektrisch mit einem an dem Griff montierten Schalter verbinden,
wobei optional der magnetische Freigabemechanismus ferner umfasst:
eine Energieversorgung (94), die elektrisch mit dem Schalter verbunden ist, wobei der Schalter einen offenen Zustand, der die Energieversorgung elektrisch von dem Elektromagneten trennt, und einen geschlossenen Zustand, der die Energieversorgung elektrisch mit dem Elektromagneten verbindet, aufweist.

15. Katheter nach einem vorhergehenden Anspruch, wobei der Katheter sterilisiert ist.

## Revendications

1. Cathéter (10) pour délivrer un implant (14) à un patient, le cathéter comprenant :
un arbre (28) définissant une lumière (30) s'étendant depuis une extrémité proximale (10A) jusqu'à une extrémité distale (10B) de l'arbre, l'arbre comprenant :
une paroi d'espacement (38) enfermant la lumière à l'intérieur d'une région distale (10C) de l'arbre, la région distale s'étendant de l'extrémité distale vers l'extrémité proximale sur une distance égale à cinq diamètres de l'arbre ; et
un mécanisme de libération magnétique (12) comprenant : un aimant (36) disposé à l'intérieur de la lumière, l'aimant étant disposé à l'intérieur de la région distale de la lumière entre la paroi d'espacement et l'extrémité proximale.

2. Cathéter selon la revendication 1, comprenant en outre :
une bande de marquage (42) circonscrivant une partie extérieure de la lumière et s'alignant avec la paroi d'espacement.

3. Cathéter selon la revendication 1 ou la revendication 2, la paroi d'espacement étant espacée de l'extrémité distale pour former une douille (40) ouverte vers l'extrémité distale de l'arbre.

4. Cathéter selon la revendication 3, comprenant en outre :
un implant (14) reçu au moins partiellement à l'intérieur de la douille.

5. Cathéter selon la revendication 4, l'implant comprenant un fil d'ancrage hélicoïdal (48) s'étendant à partir d'une surface extérieure de l'implant opposée à une surface intérieure de l'implant en butée contre la paroi d'espacement.

6. Cathéter selon la revendication 4, l'implant étant restreint en rotation à l'intérieur de la douille par un champ magnétique de l'aimant.

7. Cathéter selon la revendication 6, un axe magnétique de l'aimant étant perpendiculaire à une ligne centrale (44) de l'arbre, et un couple magnétique maximal imposé à l'implant n'imposant pas plus qu'une force de seuil sur l'implant, la force de seuil étant comprise entre 5 Newtons et 30 Newtons.

8. Cathéter selon l'une quelconque des revendications précédentes, le mécanisme de libération magnétique comprenant en outre :
un arbre interne (34) relié à l'aimant et s'étendant à l'intérieur de la lumière à partir de l'aimant jusqu'à l'extrémité proximale, l'aimant étant un aimant permanent.

9. Cathéter selon la revendication 8, comprenant en outre :
une poignée (16) ; et
une bague (72) fixée à la poignée et à l'arbre au niveau de l'extrémité proximale, la poignée restreignant le déplacement linéaire de la bague et de l'arbre par rapport à la poignée, et l'arbre pouvant tourner par rapport à la poignée autour d'une ligne centrale de la poignée.

10. Cathéter selon la revendication 9, comprenant en outre :
un bouton (20) relié à la poignée ; et
un élément terminal (24) fixé à l'arbre intérieur et coïncidant avec la poignée, les filetages externes (74A) de l'élément terminal s'engageant dans les filetages internes (74B) du bouton de telle sorte que la rotation du bouton déplace l'aimant à l'intérieur de la lumière par rapport à la paroi d'espacement.

11. Cathéter selon la revendication 10, comprenant en outre :
un capuchon d'extrémité (18) relié à la poignée opposée au bouton ; et
un ressort (22) comprimé à l'intérieur de la poignée entre le capuchon d'extrémité et l'élément terminal.

12. Cathéter selon l'une quelconque des revendications précédentes, comprenant en outre :
une gaine (32) entourant l'arbre et s'étendant de l'extrémité proximale à une extrémité terminale (32A) espacée de l'extrémité distale de l'arbre, l'arbre faisant saillie de la gaine au niveau de l'extrémité distale.

13. Cathéter selon la revendication 12, lorsque la revendication 12 dépend selon l'une quelconque des revendications 1 à 6, comprenant en outre :
une poignée (16) fixée à l'arbre au niveau de l'extrémité proximale, la poignée restreignant le déplacement linéaire et rotatif de l'arbre par rapport à la poignée.

14. Cathéter selon la revendication 13, l'aimant étant un électroaimant, l'électroaimant comprenant :
un noyau (120) ;
un enroulement (122) entourant le noyau ; et
des conducteurs (102) s'étendant des enroulements jusqu'à la poignée connectant électriquement l'électroaimant à un commutateur monté sur la poignée,
éventuellement, le mécanisme de libération magnétique comprenant en outre :
une alimentation électrique (94) connectée électriquement au commutateur, le commutateur comprenant un état ouvert qui déconnecte électriquement l'alimentation électrique de l'électroaimant et un état fermé qui connecte électriquement l'alimentation électrique à l'électroaimant.

15. Cathéter selon l'une quelconque des revendications précédentes, le cathéter étant stérilisé.
